# EUROPEAN PATENT SPECIFICATION

(11) **EP 3 160 313 B1**
(45) Date of publication and mention of the grant of the patent: **28.11.2018**
(21) Application number: 15736419.1
(22) Date of filing: 29.06.2015
(51) Int. Cl.: A23L 3/18, B08B 9/027, A47J 31/44

(54) **A SYSTEM HAVING IMPROVED RUNNING TIME**
SYSTEM MIT VERBESSERTER LAUFZEIT
SYSTÈME PRÉSENTANT UN TEMPS D'EXÉCUTION AMÉLIORÉ

(30) Priority: 30.06.2014 SE 1450807
(43) Date of publication of application: 03.05.2017
(73) Proprietor: Tetra Laval Holdings & Finance SA, 1009 Pully (CH)
(72) Inventor: OLSSON, Bo, S-21580 Malmö (SE); ANDERSSON, Göran, S-243 72 Tjörnarp (SE)
(74) Representative: Tetra Pak - Patent Attorneys SE
(86) International application number: PCT/EP2015/064708
(87) International publication number: WO 2016/001145

(56) References cited:
- GB-A- 1 125 111
- US-A- 5 755 155

## Description

### Technical Field

The invention generally relates to the field of liquid or semi-liquid food processing systems. More particularly, it is presented a system providing less risk of fouling, in turn implying increased running time.

### Background of the invention

Today, it is in the interest of liquid food processing companies around the world to make sure that the food they are processing are done so in a way that assure that the food are safe to consume. This shows for instance in that each batch is carefully followed by using different kinds of sensors and sophisticated software tools, but also in that the design of the different components in the food processing lines are made in a way such that the risk that food residues are left from one batch to another is reduced to a very low likelihood.

Just as food safety is on top of the agenda for food processing companies, it is one of their top interests to make sure that product losses are kept at a minimum. Since the raw material, such as milk or fruit, in many cases has a significant role in the operational costs, lowering the product losses have in most cases a direct effect on the financial result. Secondly, in order to make sure that the processing is environmental friendly, the effect on e.g. carbon dioxide emissions can be significantly reduced if the losses of final product can be lowered.

One reason for having product losses is because the system needs to be cleaned at regular intervals and for each cleaning session part of the product in the system when switching from production to cleaning will end up in the drain. Therefore, if the running time for the system can be increased, product losses can be reduced.

Some relevant prior art is reflected by patent documents GB1125111A and US5755155A.

### Summary

Accordingly, the present invention preferably seeks to mitigate, alleviate or eliminate one or more of the above-identified deficiencies in the art and disadvantages singly or in any combination and solves at least the above mentioned problems.

According to a first aspect it is provided a system according to claim 1.

The sterile air may be below 100 degrees C such that a part of said first body adjacent to said second body is prevented from being heated by said sterile air.

The system may further comprise a tank, wherein one and the same sterile air production apparatus is arranged to provide sterile air to said tank and said second body.

A steam inlet is arranged to provide steam into said second body, wherein said sterile air inlet is arranged closer to said first end than said steam inlet. Thereby providing for that that sterile air can be provided in the part of the second body being closest to the first body.

The steam may be 110 degrees C or higher.

A temperature sensor may be arranged to measure a temperature in said first end of said second body.

The sterile air may be produced using a sterile air filter.

The sterile air filter may be pre-sterilized at about 130 degrees C.

According to a second aspect it is provided a method according to claim 8 for controlling a system.

### Brief description of the drawings

The above, as well as additional objects, features and advantages of the present invention, will be better understood through the following illustrative and non-limiting detailed description of preferred embodiments of the present invention, with reference to the appended drawings, wherein:
Fig 1 generally illustrates an example of a downstream part of a food processing system including inter alia a tank and a filling machine.
Fig 2 generally illustrates an example of a downstream part of a food processing system including inter alia a tank and a filling machine, wherein said downstream part is provided with a circular connection.
Fig 3 is a general flow diagram illustrating another example of a downstream part of a food processing system including inter alia a tank and a filling machine, wherein said downstream part is provided with a circular connection.
Fig 4 is a general illustration of a first pipe with a second pipe connected thereto, wherein steam is provided in the second pipe.
Fig 5 is a general illustration of a first pipe with a second pipe connected thereto, wherein sterile air is provided in the second pipe.
Fig 6 is a general illustration of a first pipe with a second pipe connected thereto, wherein sterile air and steam is provided in the second pipe.

### Detailed description of preferred embodiments

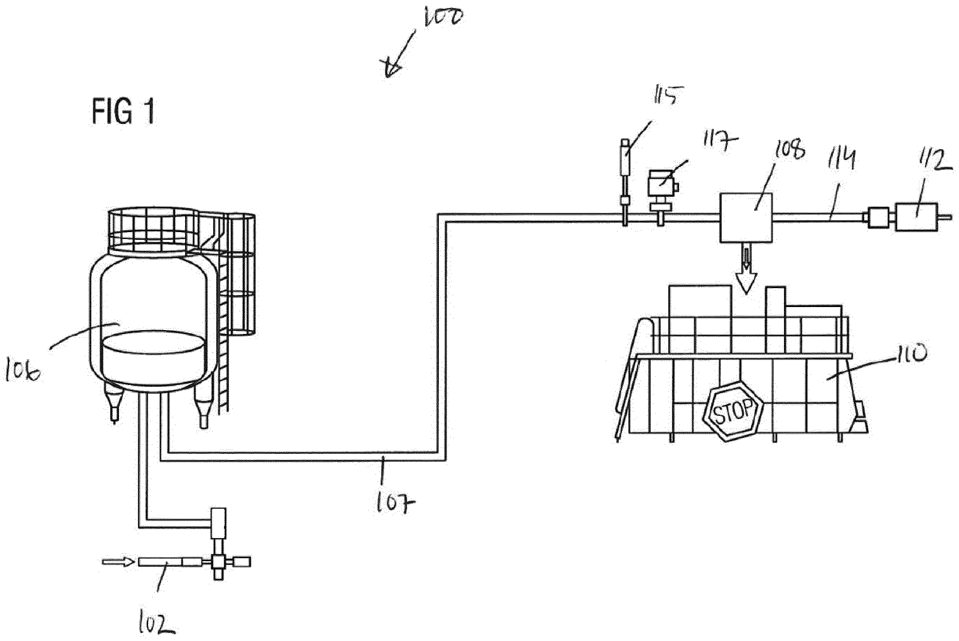

Fig 1 generally illustrates an example of a system 100 being part of a bigger system for processing and packing liquid or semi-liquid food products, such as so-called Extended Shelf Life (ESL) milk packed in carton packages. More particularly, the system 100 illustrates an inlet 102 for receiving heat treated product and feeding it to a tank 106, preferably a closed tank with a cooling system, arranged to hold the product without the risk of having it recontaminated. From the tank 106 the product is fed via a pipe 107 to a filling valve 108. The filling valve 108 can either direct the product to a filling machine 110 or to a reclaim tank via a reclaim valve 112 and a reclaim pipe 114, connecting the filling valve 108 and the reclaim valve 112.

During preparation of the system 100 product is fed via the inlet 102 to the tank 106 to form a buffer of product in the tank to balance out minor variations in the product flow upstream and downstream the tank 106. When having the buffer formed in the tank, the pipe 107 connecting the tank and the filling valve 108 is filled with product, and also the reclaim pipe 114.

After having filled the system 100 with product and it is made sure that temperature and pressure are within target intervals, for example by using a temperature sensor 115 and a pressure sensor 117, product is fed to the filling machine 110 where it is filled in packages.

During production, or in other words after having filled the system 100 and the product is fed to the filling machine 110, when handling ESL milk, the product should be kept at a temperature of about 4 degrees C to make sure that microbiological growth is prevented. If no product is fed to the reclaim tank for some time there is a risk that the product in the reclaim pipe 114 is heated, for instance by the surrounding environment, to a temperature above 4 degrees C. If this temperature increase is not prevented and kept under control, this may lead to microbacterial growth in the product kept in the reclaim pipe 114.

Further, if there is an unplanned stop of the filling machine, product will be standing in the pipe 107 connecting the tank 106 to the filling valve 108. If the stop lasts for a long period of time there is a risk of microbiological growth and, as an effect, that the product in the pipe 107 needs to be wasted and that the system needs to be cleaned before starting up again after the unplanned stop caused by the filling machine.

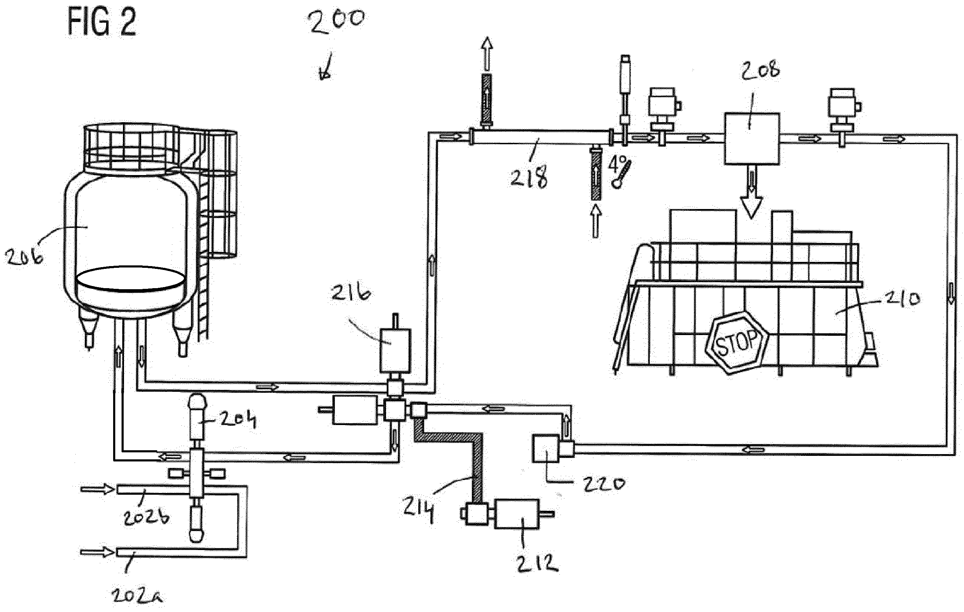

In order to overcome the problem of having product standing in the pipe 107 that may become bad in case of a long hault due to an unplanned stop caused by the filling machine 110, a system 200, illustrated in fig 2, can be used. Unlike the system 100 illustrated in fig 1, the system 200 provides for that the product can be circulated such that there will be no standing product in case of a filling machine stop.

The system 200 can comprise an inlet in turn comprising a first inlet 202a and a second inlet 202b, a valve 204 connected to to the inlet, a tank 206 forming a buffer to balance out variations in the product flow upstream and downstream, a filling valve 208 feeding part of a product flow to a filling machine 210 or in case the filling machine stops providing for that the product flow is fed pass the filling machine, a reclaim valve 212, a reclaim pipe 214 connecting the reclaim valve 212 to a valve arrangement 216, and an inline cooler 218 providing for that the product being circulated is kept within set temperature intervals.

During preparation sterile water is fed via the inlet. After having filled the system with sterile water and circulated the sterile water it can be fed out of the system to the drain via the reclaim valve 212.

If ESL milk or any other product that should be kept chilled is to be processed, the sterile water may be cold in order to make sure that the system is not only flushed, but also cooled before production starts.

After having had the system 200 flushed with sterile water, sterile air can be introduced into the system 200. Sterile air may be introduced via the tank 206 such that the sterile water present in the system is removed. This may for instance be done by introducing sterile air into the tank 206 and from there via the valve 204 to the valve arrangement 216 at the same time as sterile air is fed from the tank 206 via the valve arrangement 216, the inline cooler 218, the filling valve 208, the pump 220, back to the the valve arrangement 216, via the reclaim valve 212 to the reclaim tank.

After having flushed the system 200 with sterile water and sterile air, the system can be filled with product. This can be done by filling via the inlets 202a, 202b and feeding it via the valve 204, the tank 206, the valve arrangement 216, the inline cooler 218, the filling valve 208, the pump 220, the valve arrangement 216, the reclaim pipe 214, the reclaim valve 212 to the drain.

During production, product is fed via the inlets 202a, 202b via the valve 204, the tank 206, the valve arrangement 216, the inline cooler 218, the filling valve 208 to the filling machine 210. In order to make sure that no product is standing in the pipe connecting the filling valve 208 and the valve 204 part of the product may be diverted to the filling machine 210 and part of it circulated by feeding it back to the valve 204.

In case of an unplanned stop of the filling machine 210, or planned stop, the filling valve 208 can be changed such that all product are circulated and the incoming product result in that more product is buffered in the tank.

In order to make sure that the product is kept chilled, or in any other way within a target temperature interval, in case of a stop of the filing machine 210, the inline cooler 218 can be used. In the illustrated example, the inline cooler 218 is a tubular heat exchanger being provided with 4 degrees C water to make sure that a temperture of the product when passing the inline cooler is lowered to the target temperature interval. By doing so, the risk of having the product recontaminated during a stop of the filling machine 210 is reduced, which is positive from both a food safety perspective as well as a product loss perspective.

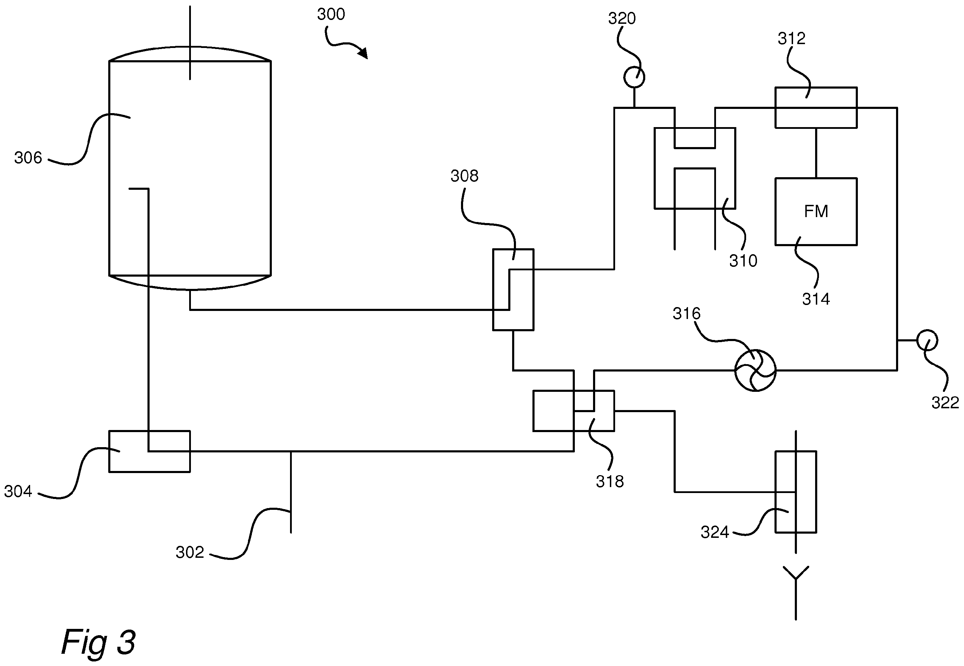

Fig 3 illustrates a flow diagram of a system 300 of a food processing system similar to the the system illustrated in fig 1 and 2.

In the system 300 liquid product can be fed via an inlet 302 via a first valve 304 to a tank 306. From the tank the liquid product can be fed via a second valve 308 and an inline cooler 310 to a diversion valve 312. The diversion valve 312 may be set to either divert part of a product flow to a filling machine 314 or not divert the flow to the filling machine such that the product flow is fed pass the filling machine 314. During production, the diversion valve can be set to divert part of the product flow. The reason why only part of the product flow is fed to the filling machine 314 is to make sure that there are no dead ends with standing product.

The product not diverted to the filling machine can be fed to a third valve 318 via a pump 316. From the third valve 318 the product may be fed to the inlet 302 such that the circular connection is achieved, providing for that the product can be circulated in the system. By having the inline cooler 310 in the system the product, when being recirculated, can be kept cool providing for that the risk for microbacterial growth can be kept low.

In order to keep track of the level in the system level sensors can be used. In the example illustrated in fig 3 a first level sensor 320 can be provided upstream the diversion valve 312 and a second level sensor 322 can be provided downstream the diversion valve 312.

The third valve 318 can be configured to either direct the product flow to the inlet as described above, or to direct the flow to a drain or reclaim system via a fifth valve 324. During cleaning, water and cleaning solutions can be sent to the drain or reclaim system.

After cleaning the fifth valve 324 may be set such that sterile air can be fed into the system via the fifth valve 324. In order to avoid that product is entering the part of the system between the fifth valve 324 and the third valve 318, herein referred to as a reclaim pipe, this part may be filled with overpressurized sterile air.

Sterile air may be used for emptying the tank 306. In case there is a system for providing sterile air to the tank this system may also be used for providing sterile air to the reclaim pipe.

An advantage of using overpressurized sterile air, that is having sterile air at a pressure in the reclaim pipe higher than a pressure in a closely placed pipe containing product during production, is that there is no need to use steam to ensure food safety. Unlike sterile air, steam will namely have the negative effect that product can burn on a part of the third valve being close to the reclaim pipe. Therefore, by having overpressurized sterile air instead of steam less fouling may occur, in turn resulting in improved running times.

When filling an empty system there may be air entrapped in the system. In order to make sure that this air is pushed out of the system and for instance not into the filling machine, the system can be filled with product by closing the first valve 304 and feeding in product via the inlet in a direction opposite to the direction of the product flow during production, namely via the third valve 318, the pump 316, the diversion valve 312, the second valve 308 to the tank 306. By using for instance a level switch in the tank 306, the system can be informed, by using a controller or other data handling apparatus, when the tank is filled to a certain level.

When the tank is filled to the certain level the first valve 304 can be opened such that product is fed from the inlet 302 via the first valve 304 to the tank 306.

In the illustrated example a first flow path from the inlet to the tank via the first valve is shorter than a second flow path from the inlet via the third valve, the pump, the diversion valve, the inline cooler, the second valve to the tank. Therefore, in the illustrated example, due to that the first flow path is shorter than the second flow path, and that the second flow path is filled with product and the first flow path is empty, with the exception of the part between the inlet and the first valve, the product flow will choose the first flow path when opening the first valve. However, if needed to direct the product fed in via the inlet to the first flow path, a valve may be provided on the second flow path close to the inlet such that the second flow path can be closed.

After having filled the first flow path with product, product can be continued to be fed into the system via the inlet and the product flow from the tank can now switch to going from the tank via the second valve 308, the inline cooler 310 to the diversion valve 312, where part of the product flow can be fed to the filling machine 314 and part be fed via the pump 316, the third valve 318 and the first valve 304 to the tank 306.

In order to make sure that product losses can be kept low the system may be emptied by pushing out product present in the system by sterile air fed into the system via the tank 306.

In a first step of an emptying process, product placed in the system beween the tank, the second valve, the inline cooler and the diversion valve 312 can be pushed out to the filling machine by using sterile air fed into the system via the tank. In order to make sure that product is fed in this direction, the first valve 304 may be closed in this first step. Another option is, if having a tank inlet placed above a tank outlet, is to have the first valve closed when the tank level is above the tank inlet.

In a second step product placed in the system between the third valve 318, the pump 316 and the diversion valve 312 can be pushed out to the filling machine 314 by setting the second valve 308 such that sterile air fed from the tank is directed to the third valve 318. By emptying part of the system backwards in this way the product placed between the third valve and the diversion valve can be packed and used, instead of being sent to the drain. Since the distance between the diversion valve and the third valve may at some sites be long substantial savings can be made.

By having the first level sensor 320, an amount of product upstream the diversion valve 312 can be measured. When the amount of product is below a threshold the system can change such that product placed between the third valve 318 and the diversion valve 312 is pushed towards the diversion valve 312 by changing the second valve 308, as explained above.

Similarly, by having the second level sensor 322, an amount of product downstream the diversion valve can be measured in order to know when to stop feeding in sterile air in order to push product towards the diversion valve.

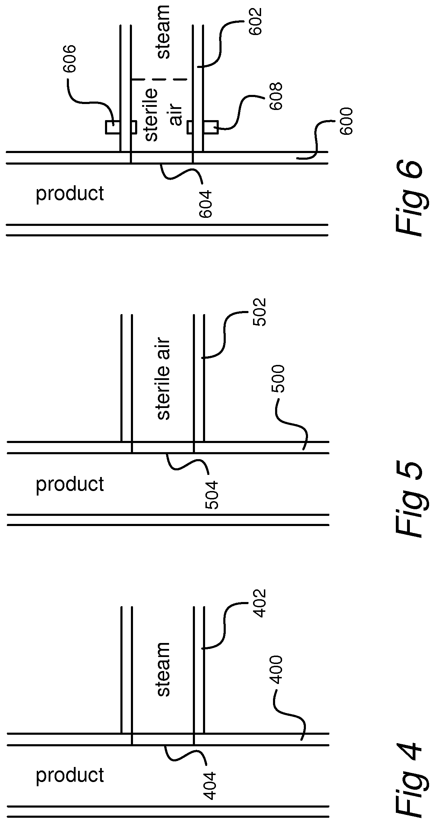

Fig 4 is a general illustration of a first pipe 400 with a second pipe 402 connected thereto. In order to avoid that product residue will migrate from the second pipe to the first pipe steam is provided in the second pipe.

Even though not illustrated as a valve, the first pipe 400 is connected to the second pipe 402 via valve, such as the third valve 318 illustrated in fig 3. In this case, the first pipe 400 would correspond to a pipe connecting the pump 316 and the inlet 302, and the second pipe 402 would correspond to the reclaim pipe connecting the third valve 318 and the fifth valve 324. This also applies to arrangements illustrated in fig 5 and 6.

In order to avoid that product residue migrate from the second pipe 402 to the first pipe 400 steam may be provided in the second pipe. An advantage of using steam is that because of the high temperature of the steam unwanted microorganisms will be killed and hence the risk of infecting the product via the second pipe in this way kept low. However, a disadvantage with using steam is that an element 404, being a shared element between the first and the second pipe, will be heated by the steam. This in turn will result in that fouling may occur on the element 404 due to the heat generated by the steam, but also that the product is heated, which may lead to micro bacterial growth. This may for instance be a problem for so-called Extended Shelf Life (ESL) milk.

In order to avoid that the product in the first pipe is heated by steam provided in the second pipe, a system as illustrated, by example, in fig 5 can be used. Fig 5 is, in the same manner as an arrangement illustrated in fig 4, a general illustration of a first pipe 500 with a second pipe 502 connected thereto. However, in order to avoid that product residue will migrate from the second pipe to the first pipe sterile air, instead of steam, is provided in the second pipe. By using sterile air the unwanted heating of an element 504 can be avoided, with the positive effects that less fouling will occur and that the product is not heated in the same manner.

In order to decrease the risk that product residues migrate from the first pipe 500 to the second pipe 502 the pressure in the second pipe is higher than the pressure in the first pipe resulting in that in case there is a leakage product will with a low likelihood be able to migrate from the first pipe to the second pipe. Since the second pipe may constitute a dead end during production this in turn provides for that there is low likelihood for microbacterial growth in the second pipe that at a later stage may migrate to other parts of the system, such as the first pipe.

Fig 6 illustrates a further embodiment. A first pipe 600 is connected to a second pipe 602 in a similar manner as illustrated in fig 4 and 5. As in fig 5, an element 604 shared between the first pipe and the second pipe is prevented from being heated by using sterile air in an end of the second pipe close to the element 604. However, in order to further reduce the risk of having microbacterial growth sterile air barrier close to the element is complemented with a steam barrier.

In order to make sure that the steam barrier does not heat the element 604 a temperature sensor 606 may be used. If it is detected by the temperature sensor 606 that the temperature in the end of the second pipe is too high, that is, there is a risk that the element will be heated with fouling and heated product as a possible outcome, sterile air may be fed into the end of the second pipe via a sterile air inlet 608.

Although not mentioned, instead of having product in the first pipe illustrated in fig 4, 5 and 5, the product may be in a tank or any other container for holding a product.

The invention has mainly been described above with reference to a few embodiments. However, as is readily appreciated by a person skilled in the art, other embodiments than the ones disclosed above are equally possible within the scope of the invention, as defined by the appended patent claims.

## Claims

1. A system comprising
a first body (600) arranged to hold liquid food product,
a second body (602) arranged to hold steam, wherein a first end of said second body (602) is adjacent to said first body (600), and
a sterile air inlet (608) arranged to provide sterile air into said first end of said second body (602),
said first body (600) and said second body (602) being connected to each other by a valve, wherein said system is arranged to provide the sterile air via said sterile air inlet (608) to said second body (602) such that a pressure in said second body (602) is greater than a pressure in said first body (600), to thereby reduce fouling, and
further comprising a steam inlet arranged to provide steam into said second body (602), wherein said sterile air inlet (608) is arranged closer to said first end than said steam inlet.

2. The system according to claim 1, wherein said sterile air is below 100 degrees C such that a part of said first body (600) adjacent to said second body (602) is prevented from being heated by said sterile air.

3. The system according to any one of the preceding claims, further comprising a tank (306), wherein one and the same sterile air production apparatus is arranged to provide sterile air to said tank (306) and said second body (602).

4. The system according to claim 1, wherein said steam is 110 degrees C or higher.

5. The system according to any one of the preceding claims, further comprising a temperature sensor (606) arranged to measure a temperature in said first end of said second body (602).

6. The system according to any one of the preceding claims, wherein said sterile air is produced using a sterile air filter.

7. The system according to claim 6, wherein said sterile air filter is pre-sterilized at about 130 degrees C.

8. A method for controlling a system comprising a first body (600) arranged to hold liquid food product, a second body (602) arranged to hold steam, wherein a first end of said second body (602) is adjacent to said first body (600), and a sterile air inlet (608) arranged to provide sterile air into said second body (602), said first body (600) and said second body (602) being connected to each other by a valve, said method comprising providing the liquid product in said first body (600),
providing sterile air in said first end of said second body (602), wherein
said liquid product in said first body (600) is provided at a first pressure and said sterile air in said first end of said second body (602) is provided at a second pressure, wherein said second pressure is higher than said first pressure, to thereby reduce fouling,
providing steam in said second body (602),
measuring a temperature of said sterile air in said first end of said second body (602), and
providing sterile air into said second body (602) to lower said temperature if said temperature is above a threshold.

## Patentansprüche

1. System, das Folgendes umfasst:
einen ersten Körper (600), angeordnet, um ein flüssiges Lebensmittelprodukt zu halten,
einen zweiten Körper (602), angeordnet zum Halten von Dampf, wobei ein erstes Ende des zweiten Körpers (602) angrenzend an den ersten Körper (600) ist, und
einen sterilen Lufteinlass (608), angeordnet, um sterile Luft in das erste Ende des zweiten Körpers (602) einzuleiten,
wobei der erste Körper (600) und der zweite Körper (602) über ein Ventil miteinander verbunden sind, wobei das System angeordnet ist, um sterile Luft über den sterilen Lufteinlass (608) in den zweiten Körper (602) einzuleiten, sodass ein Druck in dem zweiten Körper (602) größer als ein Druck in dem ersten Körper (600) ist, um dadurch Verschmutzung zu verringern, und
ferner umfassend einen Dampfeinlass, angeordnet, um Dampf in den zweiten Körper (602) einzuleiten, wobei der sterile Lufteinlass (608) näher am ersten Ende angeordnet ist als der Dampfeinlass.

2. System nach Anspruch 1, wobei der sterile Lufteinlass unter 100 Grad C ist, sodass verhindert wird, dass sich ein an den zweiten Körper (602) angrenzender Teil des ersten Körpers (600) durch die sterile Luft erwärmt.

3. System nach einem der vorhergehenden Ansprüche, ferner umfassend einen Tank (306), wobei ein und dieselbe sterile Lufterzeugungseinrichtung angeordnet ist, um sterile Luft in den Tank (306) und den zweiten Körper (602) einzuleiten.

4. System nach Anspruch 1, wobei der Dampf bei 110 Grad C oder höher ist.

5. System nach einem der vorhergehenden Ansprüche, ferner umfassend einen Temperatursensor (606), angeordnet zum Messen einer Temperatur im ersten Ende des zweiten Körpers (602).

6. System nach einem der vorhergehenden Ansprüche, wobei die sterile Luft unter Verwendung eines sterilen Luftfilters produziert wird.

7. System nach Anspruch 6, wobei der sterile Luftfilter bei etwa 130 Grad C vorsterilisiert ist.

8. Verfahren zum Steuern eines Systems, umfassend einen ersten Körper (600), angeordnet zum Halten eines flüssigen Lebensmittelprodukts, einen zweiten Körper (602), angeordnet zum Halten von Dampf, wobei ein erstes Ende des zweiten Körpers (602) angrenzend an den ersten Körper (600) ist, und einen sterilen Lufteinlass (608), angeordnet, um sterile Luft in den zweiten Körper (602) einzuleiten, wobei der erste Körper (600) und der zweite Körper (602) über ein Ventil miteinander verbunden sind, wobei das Verfahren Folgendes umfasst:
Einleiten des flüssigen Produkts in den ersten Körper (600),
Einleiten von steriler Luft in das erste Ende des zweiten Körpers (602), wobei
das flüssige Produkt in dem ersten Körper (600) bei einem ersten Druck eingeleitet wird und die sterile Luft im ersten Ende des zweiten Körpers (602) bei einem zweiten Druck eingeleitet wird, wobei der zweite Druck hoher als der erste Druck ist, um dadurch Verschmutzung zu verringern,
Einleiten von Dampf in den zweiten Körper (602),
Messen einer Temperatur der sterilen Luft im ersten Ende des zweiten Körpers (602), und
Einleiten von steriler Luft in den zweiten Körper (602), um die Temperatur abzusenken, wenn die Temperatur über einer Schwelle ist.

## Revendications

1. Système comprenant
un premier corps (600) agencé pour contenir un produit alimentaire liquide,
un second corps (602) agencé pour contenir de la vapeur, une première extrémité dudit second corps (602) étant adjacente audit premier corps (600), et
une entrée d'air stérile (608) agencée pour fournir de l'air stérile dans ladite première extrémité dudit second corps (602),
ledit premier corps (600) et ledit second corps (602) étant reliés l'un à l'autre par une valve,
ledit système étant agencé pour fournir l'air stérile par l'intermédiaire de ladite entrée d'air stérile (608) audit second corps (602) de telle sorte qu'une pression dans ledit second corps (602) est supérieure à une pression dans ledit premier corps (600), pour ainsi réduire l'encrassement, et
comprenant, en outre, une entrée de vapeur agencée pour fournir de la vapeur dans ledit second corps (602), ladite entrée d'air stérile (608) étant agencée plus près de ladite première extrémité que ladite entrée de vapeur.

2. Système selon la revendication 1, ledit air stérile étant inférieur à 100 °C de telle sorte qu'une partie dudit premier corps (600) adjacente audit second corps (602) est empêchée d'être chauffée par ledit air stérile.

3. Système selon l'une quelconque des revendications précédentes, comprenant en outre un réservoir (306), un seul et même appareil de production d'air stérile étant agencé pour fournir de l'air stérile audit réservoir (306) et audit second corps (602).

4. Système selon la revendication 1, ladite vapeur étant à 110 °C ou plus.

5. Système selon l'une quelconque des revendications précédentes, comprenant en outre un capteur de température (606) agencé pour mesurer une température dans ladite première extrémité dudit second corps (602).

6. Système selon l'une quelconque des revendications précédentes, ledit air stérile étant produit à l'aide d'un filtre à air stérile.

7. Système selon la revendication 6, ledit filtre à air stérile étant pré-stérilisé à environ 130 °C.

8. Procédé de commande d'un système comprenant un premier corps (600) agencé pour contenir un produit alimentaire liquide, un second corps (602) agencé pour contenir de la vapeur, une première extrémité dudit second corps (602) étant adjacente audit premier corps (600), et une entrée d'air stérile (608) agencée pour fournir de l'air stérile dans ledit second corps (602), ledit premier corps (600) et ledit second corps (602) étant reliés l'un à l'autre par une valve, ledit procédé comprenant :
la fourniture du produit liquide dans ledit premier corps (600),
la fourniture d'air stérile dans ladite première extrémité dudit second corps (602),
ledit produit liquide dans ledit premier corps (600) étant fourni à une première pression et ledit air stérile dans ladite première extrémité dudit second corps (602) étant fourni à une seconde pression, ladite seconde pression étant supérieure à ladite première pression, pour ainsi réduire l'encrassement,
la fourniture de vapeur dans ledit second corps (602),
la mesure d'une température dudit air stérile dans ladite première extrémité dudit second corps (602), et
la fourniture d'air stérile dans ledit second corps (602) pour abaisser ladite température si ladite température est supérieure à un seuil.
